# EUROPEAN PATENT APPLICATION

(11) **EP 0 895 759 A1**
(43) Date of publication of application: **10.02.1999**
(21) Application number: 98114805.9
(22) Date of filing: 06.08.1998
(51) Int. Cl.: A61F 2/06

(54) **An angioplasty stent, particularly for treating aorto-ostial and ostial lesions**

(30) Priority: 08.08.1997 IT TO970728
(71) Applicant: SORIN BIOMEDICA CARDIO S.p.A., I-13040 Saluggia, Vercelli (IT)
(72) Inventor: Bartorelli, Antonio, 20121 Milano (IT); Vallana, Franco, 10123 Torino (IT)
(74) Representative: Bosotti, Luciano

(57) **Abstract**

The stent comprises a body (1) with a generally tubular envelope which can be expanded in use from a radially contracted condition to a radially expanded condition. The body of the stent comprises a segment (2) which is substantially proximal in the position of use and which has a greater resistance to radial compression from the radially expanded condition than the remaining portion (3) of the body of the stent (1).

## Description

The present invention relates to angioplasty stents.

This term defines, in general, a device to be fitted in a lumen (for example, inside a blood vessel), usually by catheterization, and subsequently spread out in situ in order to support the lumen locally. The main purpose of this support is to eliminate a stenotic site present in the location treated by preventing re-establishment of the stenosis.

For a general teaching with regard to vascular stents, reference may usefully be made to the work "Textbook of Interventional Cardiology" by Eric J. Topol., W.B. Saunders Company, 1994 (see, in particular, Section IV of Vol. II, entitled "Coronary stenting") and "Endoluminal Stenting" by Ulrich Sigwart, W.B. Saunders Company, 1996.

A very large number of patent documents is also devoted to the subject as is shown, for example, by US-A-4 776 337, US-A-4 800 882, US-A-4 907 336, US-A-4 886 062, US-A-4 830 003, US-A-4 856 516, US-A-4 768 507, US-A-4 503 569 and EP-A-0 201 466. It should, however, be pointed out that it has already been proposed in the art to use substantially similar structures for spreading out and anchoring vascular grafts in situ; naturally, this possible extension of the field of application should be understood as being included within the scope of the invention.

In spite of the extensive research and experimentation as documented at patent level, only a very small number of operative solutions has up to now been used in practice. This can be attributed to various factors, amongst which the following problems or requirements may be mentioned:
- to ensure that, during its advance towards the site to be treated, the stent can adapt in a sufficiently flexible manner to the path travelled, even in portions having small radii of curvature, and without adversely affecting the ability of the stent to perform an effective supporting action once positioned and spread out,
- to prevent, or at least limit, the effect of longitudinal shortening which occurs in some stents when they are spread out,
- to offer as extensive as possible a bearing surface to the lumen wall to be supported,
- to avoid giving rise to complex geometry and/or to possible stagnation sites which could cause problems such as coagulation, clotting, etc., and
- to reconcile the requirements set out above with simple and reliable production methods and criteria within the scope of currently-available technology.

The solutions proposed in the art up to now have been directed in general towards achieving behaviour which is as uniform as possible throughout the stent wall. This relates both to the radial expansion which it is generally desired to achieve substantially isodynamically around the entire periphery of the stent, and with regard to flexibility and resistance to relaxation (so-called "recoil") measured longitudinally of the stent.

These considerations also apply to the stent sold under the trade mark Jostent by Jomed International AB of Helsingborg (Sweden), in which there is at least one portion characterized by a mesh structure with dimensions approximately double those in the remaining portion of the stent. The advertising literature relating to this stent in fact states that the composition and processing of the material used to produce it provide a high degree of radial strength distributed uniformly over the entire length of the stent. In particular, the region of the stent in question in which the large-mesh structure is present is intended to be located in the region of a bifurcation, and hence in a region in which secure support of the walls of the vessels concerned is required.

The development of angioplasty techniques for coronary regions has demonstrated the importance of an ability to intervene effectively on aorto-ostial and ostial lesions.

In this context of use, the structural components of the artery walls concerned contribute significantly - by acting in practice as a kind of sphincter - to the stressing of the stent wall in the sense of a radial relaxation of its structure.

As a first obvious reaction, this would lead to the use, for the treatment of these sites, of stents having marked characteristics of resistance to radial compression from the radially extended or expanded condition.

However, this selection has been found to conflict with two other operative and applicational requirements, that is:
- the requirement to provide a stent which in any case has a certain degree of flexural resilience so that it can adapt easily to the longitudinal anatomical shape of the vessel treated, and
- the requirement to avoid the need to apply large mechanical stresses for spreading out the stent with a resulting risk of rupture of the balloon catheter usually used for this operation.

The object of the present invention, which has the specific characteristics recited in the following claims, is to solve - in addition to the general problems inherent in the production of angioplasty stents - also the specific problems outlined above with specific reference to the treatment of aorto-ostial and ostial vessels.

The invention will now be described purely by way of non-limiting example, with reference to the appended drawings, in which:
Figure 1 shows schematically the treatment of aorto-ostial and ostial lesions by means of stents according to the invention,
Figure 2 is a side view of an angioplasty stent formed in accordance with the invention,
Figure 3 is generally comparable to Figure 2 and shows a variant of the stent according to the invention, and
Figure 4 is a graph showing two possible characteristic curves of the resistance of a stent according to the invention to radial compression.

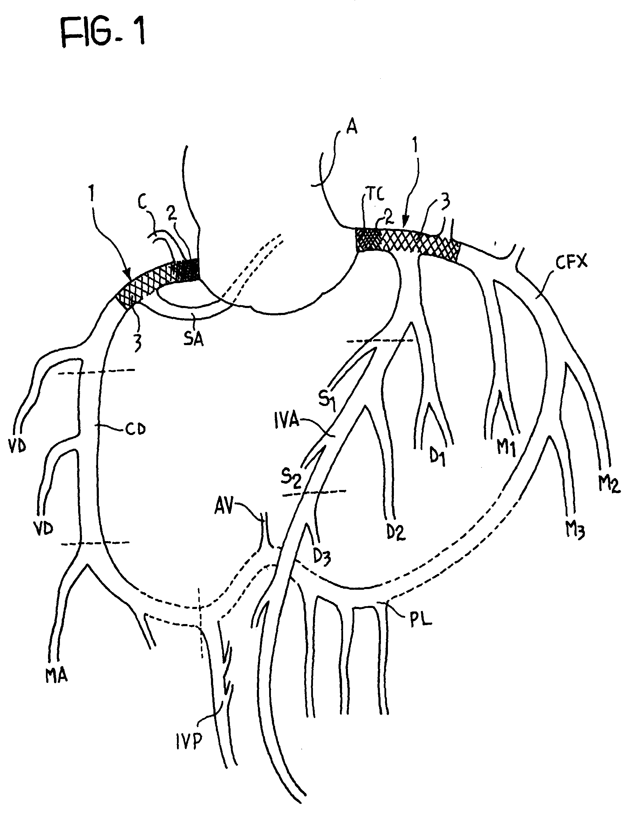

Figure 1 shows the network of coronary vessels in the typical conventional format of the sheets used for coronarography reports.

In particular, the symbols given in Figure 1 identify the following vessels:
- A:: aorta
- CD:: right coronary artery
- C:: conus branch
- SA:: atrial sinus branch
- VD:: right anterior ventricular branches
- MA:: acute marginal branch
- IVP:: posterior interventricular artery
- AV:: atrio-ventricular node branch
- TC:: left common coronary trunk
- IVA:: anterior interventricular artery
- S1, S2:: first and second anterior septal branches
- D1, D2, D3:: first, second and third diagonal branches
- CFX:: circumflex artery
- M1, M2, M3:: first, second and third marginal branches of the circumflex artery
- PL:: postero-lateral branches.

Figure 1 also shows, by way of example, two possible methods of angioplasty intervention implemented by means of stents, respectively:
- in the proximal portion of the right coronary artery CD, and/or
- in the common trunk TC of the left coronary artery.

It is quite clear that the methods of intervention shown are purely examples since the treatment may also relate merely to one of the aorto-ostial sites shown and/or to any ostial site such as, for example, the ostium of the anterior interventricular artery (IVA in Figure 1).

It has also been assumed, purely by way of example, that the extent of the lesions treated (and hence the length of the stents used) is such as also to involve some bifurcations such as the branching-off of the conus branch C and of the atrial sinus branch SA (in the case of the right coronary artery) or of the anterior interventricular artery IVA (in the case of the left coronary artery).

These particular methods of intervention mean that it is considered preferable to use a stent which, in addition to the characteristics described specifically in the present application, also has the characteristics described in another patent application filed by the Application on the same date.

In any case, the selection of particular values of the length of the stent in dependence on the specific methods of treatment envisaged constitutes a variable which may be used freely in the solution according to the invention.

In summary (and with reference again to the documentation mentioned in the introductory portion of the description for a general definition of the methods of use and of the structural features), the stent 1 is usually produced in the form of a body with a tubular envelope having an overall length of between a few millimetres and a few tens of millimetres. The body has a wall thickness (the wall usually being of a mesh or loop structure with openings, as will be explained further below) of the order, for example, of approximately 70-140 microns, in view of its possible implantation in a site in which a stenosis is to be remedied. The stent is normally located in situ by catheterization with subsequent radial expansion from an insertion diameter of the order, for example of approximately 1-1.6 mm to an expanded diameter, for example, of the order of approximately 3-5 mm so that, in the expanded condition, the stent supports the lumen, preventing recurrence of a stenosis. In general, the outside diameter in the radially contracted condition is selected so as to allow the stent to be introduced into the lumen being treated, whereas the expanded diameter corresponds, in general, to the diameter to be maintained and established in the lumen once the stenosis has been eliminated.

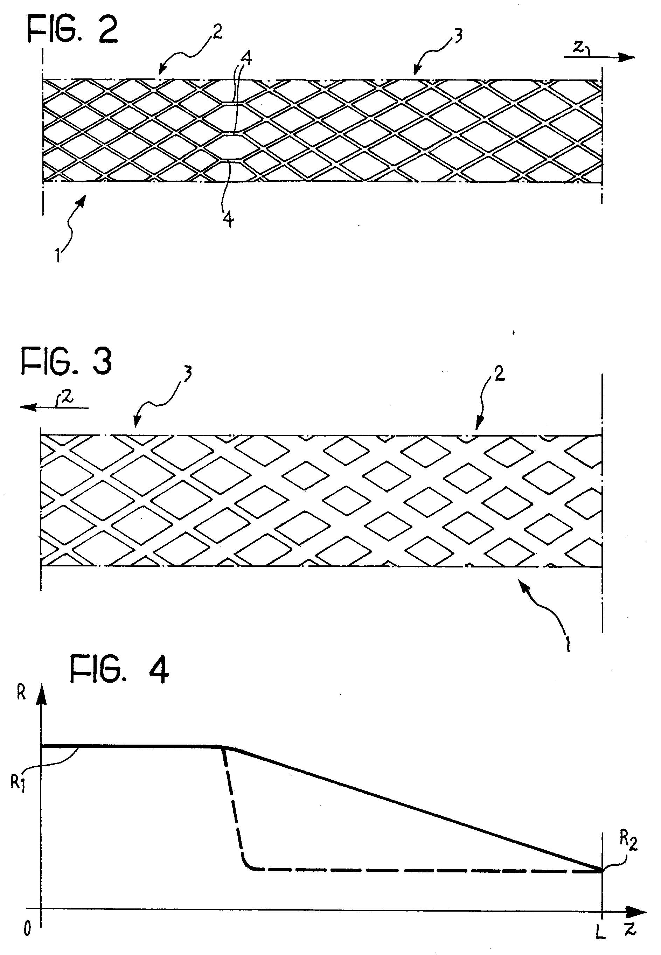

The stents 1 shown in Figures 2 and 3 are illustrated in the radially expanded condition since, in this condition, it is easier to show the characteristics described and claimed.

In order to spread out the stent in situ, the solution most generally used provides for the use of a balloon catheter, the stent being disposed around the balloon of the catheter in the contracted condition and the balloon then being expanded once the stent has been brought to the site in which it is to be positioned. However, other solutions are possible, for example, the use of superelastic materials which cause the stent to expand once the restraining elements which are intended to keep the stent in the contracted condition until the implant site has been reached, are removed. In addition or alternatively, materials having a so-called "shape memory" may also be used to form the stent so as to achieve the radial expansion in the implant position.

Usually (for more precise indications, reference should be made to the bibliographical and patent documentation cited in the introduction to the description), the stent is made of metal which can reconcile two basic requirements of the application, that is, plastic deformability during the expansion stage and the ability to withstand any stresses which would tend to cause the stent to close up again, preserving the expanded shape.

In any case, these technological aspects will not be dealt with in detail in the present description since they are not relevant *per se* for the purposes of understanding and implementing the invention. This also applies basically to the technology for the production of the stents according to the invention. As already stated, in general terms, these adopt the appearance of bodies with tubular envelopes having walls with openings. With regard to the production methods, according to the prior art, at least three basic solutions may be used, that is:
- producing the stent from a continuous tubular blank to be cut up into individual stents, the portions with openings being formed by techniques such as laser-cutting, photo-engraving, electron-discharge machining, etc;
- producing the stent from a strip-like body in which the regions with openings are formed, for example, by the techniques mentioned above, with a view to the subsequent closure of the strip-like element to form a tube, and
- producing the stent from shaped metal wire with subsequent connection of the loops of wire, for example, by micro-welding, brazing, gluing, crimping, etc.

The first solution described is that which is currently preferred by the Applicant for producing the stents in accordance with the embodiments described below.

In any case, this production aspect is not relevant *per se* for the purposes of the implementation of the invention. This also applies with regard to the selection of the individual techniques and of the order in which the various steps described (the production of the portions with openings, parting, any bending of the strip-like element, etc.) are carried out.

In all of the embodiments described herein, the body of the stent 1 extends in a longitudinal direction generally indicated by an axis z. For simplicity of illustration, reference is made herein, purely by way of non-limiting example, to a stent 1 the wall of which has a generally rhombic mesh structure. It will, however, be appreciated that the invention may also be implemented in stents 1 having any wall geometry such as, for example, the geometries described in the previous patent applications relating to angioplasty stents filed by the Applicant. This applies, in particular but not exclusively, to the mesh-like geometry described in European patent application No. 98107382.8.

Both in the case of the embodiment shown in Figure 2 and in the case of the embodiment shown in Figure 3, a stent 1 according to the invention is characterized by the presence of a first portion or segment 2 (the proximal or substantially proximal segment in the implanted condition of the stent) which, in comparison with the remaining (generally distal) portion 3 of the stent, has a substantially more marked stiffness or resistance to radial compression stresses. In other words, the stresses referred to are those which, when the stent 1 is in the implanted position, would theoretically tend to cause its structure to yield in the sense of a more or less marked relaxation in a radial direction (a phenomenon generally known as "recoil"). This phenomenon may have acute consequences such as endoluminal thrombosis and, in the medium term, re-stenosis of the site treated.

The attribute "proximal or substantially proximal" applied to the segment 2 refers to the condition of implantation shown schematically in Figure 1. In fact it can be seen that, in both of the implant situations shown, the stent 1 is implanted in a manner such that the segment 2 is in an aorto-ostial or ostial position.

Stating that the segment 2 is located - substantially - in an end position, is intended to indicate that although, in the embodiments illustrated, the segment 2 occupies an end position within the overall axial extent of the stent 1, the use should not be completely excluded of solutions in which, although the segment 2 is located in a generally lateral position relative to the overall extent of the stent, it does exactly occupy an end position since this position is occupied by a completing segment with different characteristics.

In any case, what is important for the invention is that, in the implant position, the segment 2 can be located in the region in which the wall of the stent 1 is subjected to greater stresses in the sense of a possible radial relaxation.

Tests carried out by the Applicant show that, in the treatment of aorto-ostial and ostial lesions, a wholly satisfactory restraint of these phenomena can be achieved by giving the segment 2 an overall length of the order of about 1 cm.

The remaining portion 3 of the stent which - as already stated - is disposed in a generally distal position relative to the segment 2, is formed (in known manner) so as to have characteristics of longitudinal flexibility such that it can adapt easily to the longitudinal anatomy of the vessel treated, which factor facilitates angioplasty treatment.

Figures 2 and 3 relate to two possible solutions which may be adopted in order to confer on the segment 2 the desired characteristics of greater resistance to radial compression stresses.

In the embodiment of Figure 2, this result has been achieved, for example, by the formation of the segment 2 with a denser mesh structure than the remaining portion 3 of the stent.

In other words, in the solution shown in Figure 2, the segment 2 has a greater number of meshes per unit area (of the tubular wall of the stent 1) than the portion 3. The number of meshes generally determines the number of loop portions affected by the plastic deformation of the stent during the expansion from the radially contracted position to the radially expanded position. If - for given other conditions - the number of meshes and hence the number of loops subjected to deformation is increased, the stiffness offered by the stent 1 in relation to any contraction of its radial dimensions once it is expanded, can correspondingly be increased.

In the embodiment of Figure 3, the same result has been achieved by keeping the number of meshes (and hence of loops) per unit area of the wall substantially identical both in the segment 2 and in the remaining portion 3 of the stent, but operating (in known manner, for example, during the manufacture of the stent by laser cutting) in a manner such that the openings of the meshes of the segment 2 are defined by wider wall portions or "braces", particularly in the loop regions. This results in a stiffening of those regions which are responsible for the resistance of the stent wall to deformations in the sense of a variation in its radial dimensions.

Clearly, the solutions shown in Figures 2 and 3 constitute only two of the many which may be adopted to achieve the desired result, that is, to arrange for the segment 2 of the stent to have a resistance to radial compression from the radially expanded condition substantially greater than the homologous resistance of the remaining portion 3 of the stent body.

To give another example, the same result could be achieved, with a variable degree of difficulty depending on the type of technology adopted for the production of the stent, by varying the thickness (measured radially) of the stent wall (thicker in the segment 2, thinner in the remaining portion 3), or by varying the geometry of the meshes and, in particular of the loop portions, so as to have meshes with substantially similar characteristics throughout the length of the stent, except for the loop portions which are responsible for the radial deformation characteristics.

Clearly, all of the solutions given above (variation of the number, variation of the width, variation of the thickness, variation of the geometry of the meshes and, in particular of the loop portions thereof which are responsible for the radial deformation characteristics of the stent) may be combined with one another in various ways.

The foregoing also applies with regard to the determination of the flexibility of the remaining portion 3 of the stent and its connection to the segment 2.

For example, the embodiment of Figure 2 corresponds to a stent in which the portion 3 has characteristics of flexibility (both radial and longitudinal) which are substantially uniform throughout its length.

The necessary transition with respect to the segment 2 in this case is achieved by means of a set of connecting bridges (links) 4 extending, for example, generally axially relative to the stent 1.

In the embodiment of Figure 3, the flexibility (which, in this embodiment, is linked basically to the width of the socalled "braces" which define the meshes) is modulated in the distal portion 3 of the stent so as to achieve a gradual change between the maximum level of radial stiffness (R1) defined by the segment 2 and a minimum value (R2) achieved at the opposite end of the portion 3 to the segment 2.

These two possible solutions are illustrated schematically in the graph of Figure 4. This shows the resistance of the stent to radial compression in the expanded condition (shown in arbitrary units since the relative value is more significant, for the purposes of an understanding of the invention, than the absolute value) plotted against the length (the axis z) of the stent. The corresponding abscissa value is thus between 0 which corresponds to the outer edge of the segment 2 and L which corresponds to the opposite end of the portion 3, and hence to the overall length of the stent.

In both cases, the stiffness value R adopts the uniform or substantially uniform maximum value R1 in the segment 2.

In the embodiment of Figure 2 (the broken line in the graph of Figure 4) the stiffness value R shows a rapid downward transition in the region of the links 4, bringing it, immediately afterwards, to the minimum value R2 which is maintained in a generally uniform manner throughout the portion 3 of the stent as far as its opposite end.

In the embodiment of Figure 3, however, (the solid line in the graph of Figure 4) the transition between the values R1 and R2 takes place gradually, for example, in accordance with a linear law, the values R1 (maximum) and R2 (minimum) being reached in the connecting region between the two portions 2 and 3 and at the opposite end of the segment 3 to the segment 2, respectively.

Clearly, by altering the parameters given above, it is possible to achieve, both within the segment 2 and - above all - within the remaining segment 3, a modulation of the stiffness R in accordance with practically any law, according to the specific requirements of use.

With regard to the ratio R1:R2, tests carried out by the Applicant show that the best results are achieved with values of this ratio of substantially between about 2.5:1 and 5:1 with most preferred values around 3:1. Naturally, the advantages of the invention are also appreciable with values lower than those indicated and reduce gradually as the ratio tends towards unity. Conversely, the use of higher values, for example, of the order of about 10:1 in certain fields of application cannot be excluded.

Although the present description has been given with particular reference to the treatment of aorto-ostial and ostial lesions, the solution according to the invention is suitable, in general, for angioplasty treatment of all lesions having characteristics which may give rise to substantially similar problems in treatment; the scope of the invention should not therefore be interpreted as being in any way limited to this particular example of use which is, however to be considered preferable at the moment.

Naturally, the principle of the invention remaining the same, the details of construction and forms of embodiment may be varied widely with respect to those described and illustrated, without thereby departing from the scope of the present invention.

## Claims

1. An angioplasty stent comprising a generally tubular envelope body (1) which can be expanded from a radially contracted condition to a radially expanded condition, characterized in that the body comprises a segment (2) of which the resistance (R1) to radial compression from the radially expanded condition is substantially greater than the homologous resistance (R2) of the remaining portion (3) of the stent body.

2. A stent according to Claim 1, characterized in that the segment (2) is substantially in an end position relative to the stent (1).

3. A stent according to Claim 1 or Claim 2, characterized in that the segment (2) has a length of about one centimetre.

4. A stent according to any one of Claims 1 to 3, characterized in that the ratio between the resistance (R1) and the homologous resistance (R2) is greater than about 2.5:1.

5. A stent according to Claim 4, characterized in that the ratio is between about 2.5:1 and about 5:1.

6. A stent according to Claim 5, characterized in that the ratio is substantially equal to about 3:1.

7. A stent according to Claim 4, characterized in that the ratio is less than about 10:1.

8. A stent according to any one of Claims 1 to 7, characterized in that the resistance (R1) is substantially uniform throughout the segment (2).

9. A stent according to any one of Claims 1 to 8, characterized in that the homologous resistance (R2) is substantially uniform throughout the remaining portion (3) of the stent.

10. A stent according to Claim 2, characterized in that the homologous resistance (R2) has a minimum value at the opposite end of the remaining portion (3) of the stent to the segment (2), and in that the homologous resistance (R2) varies continuously along the remaining portion (3) of the stent.

11. A stent according to any one of the preceding claims, characterized in that the body has a structure with openings defined by respective wall portions, and in that at least one of the resistance (R1) and the homologous resistance (R2) is determined as a result of the variation of at least one of the characteristics of the stent body selected from the group constituted by: the number of openings per unit area, the width of the wall portions, the thickness of the wall portions, the geometry of the wall portions, and combinations thereof.
